# EUROPÄISCHE PATENTSCHRIFT

(11) **EP 0 888 305 B1**
(45) Veröffentlichungstag und Bekanntmachung des Hinweises auf die Patenterteilung: **10.11.1999**
(21) Anmeldenummer: 97915376.4
(22) Anmeldetag: 17.03.1997
(51) Int. Cl.: C07D 213/61, C07D 213/42, C07D 213/38, A01N 43/40, C07B 39/00, C07B 43/04

(54) **VERFAHREN UND ZWISCHENPRODUKTE ZUR HERSTELLUNG VON PYRIDYL-4-FLUORANILINEN**
PROCESS AND INTERMEDIATE PRODUCTS FOR PREPARING PYRIDYL-4-FLUOROANILINES
PROCEDE ET PRODUITS INTERMEDIAIRES POUR PREPARER DES PYRIDYLE-4-FLUOROANILINES

(30) Priorität: 18.03.1996 DE 19610571
(43) Veröffentlichungstag der Anmeldung: 07.01.1999
(73) Patentinhaber: BASF AKTIENGESELLSCHAFT, 67056 Ludwigshafen (DE)
(72) Erfinder: HARREUS, Albrecht, D-67063 Ludwigshafen (DE); GÖTZ, Norbert, D-67547 Worms (DE); RACK, Michael, D-69123 Heidelberg (DE); ISAK, Heinz, D-67459 Böhl-Iggelheim (DE); SCHÄFER, Peter, D-67308 Ottersheim (DE)
(74) Vertreter: Kinzebach, Werner, Dr.
(86) Internationale Anmeldenummer: EP9701336
(87) Internationale Veröffentlichungsnummer: WO9734872

(56) Entgegenhaltungen:
- DE-A- 1 945 625
- DE-A- 2 455 238
- DE-A- 4 323 916
- JOURNAL OF ORGANIC CHEMISTRY, Bd. 39, Nr. 12, 1974, EASTON US, Seiten 1758-1761, XP002034217 T.B. PATRICK ET AL.: "Synthesis of fluoroaromatic amines." in der Anmeldung erwähnt

## Beschreibung

Die vorliegende Erfindung betrifft N-Phenylhydroxylaminverbindungen, ein Verfahren zur Herstellung dieser Verbindungen und ein Verfahren zur Umwandlung dieser Verbindungen in Pyridyl-4-fluoranilinverbindungen.

2-Pyridyl-4-fluoranilinverbindungen sind in der WO-A-95/02580 beschrieben. Sie entsprechen der Formel I 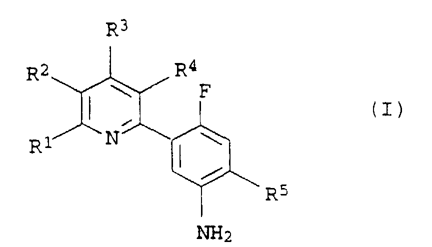 und eignen sich als Herbizide zur Defoliation und zur Desikkation. Außerdem sind sie als Zwischenprodukte zur Herstellung von Phenylpyridinen, die ebenfalls in der WO-A-95/02580 beschrieben sind, brauchbar. Die Synthese dieser Verbindungen erfolgt durch Umsetzung eines Halogenpyridins der Formel V mit einer Boronsäure der Formel VI in Gegenwart eines Übergangsmetallkatalysators nach folgendem Reaktionsschema:

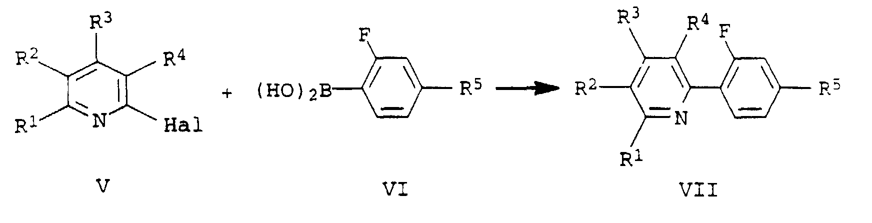

Die Verbindungen der Formel I können dann durch Nitrierung einer Verbindung der Formel VII zu einer Verbindung der Formel VIII und anschließende Reduktion gemäß folgendem Reaktionsschema erhalten werden:

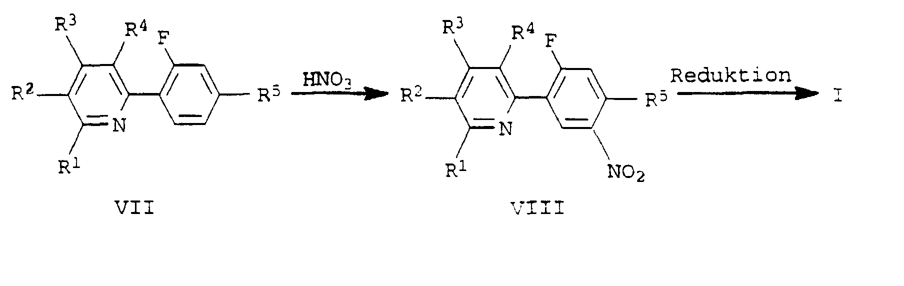

Die Herstellung der Boronsäure VI erfolgt durch Umsetzung der entsprechenden Aryllithium- bzw. Arylmagnesiumverbindungen mit Trialkylboraten und anschließende Hydrolyse.

Dieser Syntheseweg ist aber aus den folgenden Gründen nachteilig:
- Bereits in der ersten Stufe der Synthese kommen fluorhaltige Ausgangsverbindungen zur Anwendung. Diese sind in der Regel nicht einfach verfügbar, sondern müssen über eine separate, teilweise mehrstufige Synthese hergestellt werden. Zudem sind die halogenhaltigen Verbindungen teuer.
- Da in der Regel keine chemische Umsetzung quantitativ verläuft fallen im Laufe der Synthese halogenhaltige und insbesondere fluorhaltige Abfallstoffe an, deren Entsorgung problematisch sein kann.
- Ferner können Fluoratome an aromatischen Ringsystemen nucleophil substituiert werden. Dies bedingt, daß bei allen Synthesestufen solche Reaktionspartner und Reaktionsbedingungen vermieden werden müssen, die zu einer nucleophilen Substitution führen könnten. Außerdem sind Reduktionsbedingungen zu vermeiden, weil dabei eine Defluorierung eintreten kann.
- Wenn Fluoratome unter wäßrigen Bedingungen am Aromaten substituiert werden, bildet sich hochkorrosive Flußsäure, so daß die Umsetzungen in teuren Spezialapparaturen durchgeführt werden müssen.
- Schließlich muß die Herstellung der erwähnten Aryllithium-bzw. Arylmagnesiumverbindungen bei tiefen Temperaturen durchgeführt werden, weil sonst die Eliminierung von Metallfluorid unter Arinbildung störend in Erscheinung tritt oder gar zur Hauptreaktion wird.

Eine Möglichkeit zur Herstellung von 4-Fluoranilinverbindungen besteht in der Einführung des Fluoratoms in den aromatischen Kern nach der HF-Variante der Bamberger-Reaktion. Diese Variante kam bisher vor allem zur Herstellung einfacher Verbindungen, wie p-Fluoranilin, zur Anwendung, siehe Titov et al., Zh. Obshch. Khim 23, 346 (1953), US-A-4,391,991, WO-A-91/17138 und US-A-5,166,401. Darüber hinaus berichten Patrick et al. in J. Org. Chem. 39, Seiten 1758 bis 1761 (1974) über Untersuchungen an substituierten N-Phenylhydroxylaminen. In keinem Beispiel lagen ihre Ausbeuten über 61 %. Dabei wird darauf hinaewiesen, daß die Anwendungsbreite der erwähnten Bamberger-Reaktion vor allem durch die schlechte Verfügbarkeit der benötigten N-Phenylhydroxylaminverbindungen eingeschränkt ist. Ursache hierfür ist, daß es bislang keine allgemein einsetzbaren Reduktionsmethoden zur Überführung von Nitroaromaten in die N-Phenylhydroxylamine in hoher Ausbeute gibt. Patrick et al. erhielten bei ihren Reduktionen mit Zinkstaub/Ammoniumchlorid, Ammoniumsulfid oder Natriumborhydrid die gewünschten N-Phenylhydroxylamine in bestenfalls mäßigen Ausbeuten.

Um den Nachteil der schlechten Zugänglichkeit der N-Phenylhydroxylamine zu umgehen, sind eine Reihe von Umsetzungen beschrieben worden, bei denen die Reduktion der Nitroverbindungen in Gegenwart von Fluorwasserstoff vorgenommen wurde, um beide Stufen, nämlich Reduktion und Bamberger-Umlagerung, in einer Stufe zusammenzufassen (US-A-2,884,458; US-A-3,580,951; US-A-3,558,707; DE-A-19 45 625 und GB-A-2,241,952). Hieraus resultieren jedoch in der Regel Mischungen aus dem gewünschten p-Fluoranilin und dem nicht-fluorierten Anilin, das durch direkte Reduktion aus der Nitroverbindung gebildet wird, siehe beispielsweise J. Fluorine Chem. 74 (1995) 251-254. Die Trennung dieser chemisch sehr ähnlichen Verbindungen ist jedoch aufwendig und mit hohen Ausbeuteverlusten verbunden, siehe beispielsweise DE-A-1,945,625.

Eine Methode zur Herstellung von N-Phenylhydroxylaminen ist die seit langem bekannte katalytische Hydrierung von aromatischen Nitroverbindungen (Houben-Weyl "Methoden der Org. Chemie" Bd. 10/1, S. 1155 - 1157; Bd. E 16a, Teil 1, S. 49 - 53). Im Vergleich zur relativ teuren elektrochemischen Reduktion und zur Reduktion mit Metallen, wie beispielsweise mit Zinkstaub, Amalgamen u. a., die eine ungünstige Abfallstoffbilanz aufweisen, ist die katalytische Hydrierung aus wirtschaftlicher Sicht die günstigste Methode. Ein Problem stellen bei diesem Reaktionstyp die Weiterreaktion zum aromatischen Amin, der stabilen Endstufe, und die Disproportionierung des gebildeten N-Phenylhydroxylamins zu den entsprechenden Nitrosoverbindungen und Anilinen dar. Aus diesen unerwünschten Zwischen- und Folgeprodukten können durch Folgeumsetzungen höhermolekulare Nebenprodukte, wie Azoxybenzole, Azobenzole und Hydrazobenzole gebildet werden, die beispielsweise durch Kondensation von Nitrosobenzolen und N-Phenylhydroxylaminen und Weiterreaktion der gebildeten Azoxybenzole entstehen können und unter Umständen erhebliche Ausbeuteverluste verursachen.

In der Regel werden Pd- oder Pt-Katalysatoren für diese Hydrier-reaktionen empfohlen. Um Ausbeuten bzw. Selektivitäten > 50 % zu erreichen, sind nach derzeitigem Kenntnisstand Katalysatorzusätze in Form von Dimethylsulfoxid, divalenten Schwefelverbindungen oder verschiedenen organischen Phosphorverbindungen erforderlich (EP-A- 85890, EP-A- 86363, EP-A- 147879, US-A- 3694509, EP-A-212375).

Mit diesen Zusätzen wird die Verbesserung der Selektivität durch Herabsetzen der Reaktionsgeschwindigkeit erreicht, was wiederum zu langen Reaktionszeiten führt. Weiterhin hat die teilweise Vergiftung bzw. Inaktivierung des Katalysators durch die Zusätze zur Folge, daß der Katalysator meistens schon nach einem Cyclus seine Aktivität verloren hat und erneuert werden muß.

Eine weitere Methode zur Herstellung von Phenylhydroxylaminen ist die katalytische Hydrierung von Nitroaromaten in Gegenwart von organischen Stickstoffbasen, wie Piperidin, Pyrrolidin, Pyridin u.a., die -bezogen auf das Einsatzprodukt- im Überschuß eingesetzt werden müssen (DE-A- 2 455 238, DE-OS 2 455 887, DE-A- 2 357 370, DE-A- 2 327 412). Die nach diesem Verfahren erreichbaren Ausbeuten liegen nach entsprechender Aufarbeitung und Reinigung bei 80 - 85 %. Nachteilig ist, daß sich mit dieser Variante nur relativ einfache Alkyl- und Chlornitrobenzole zu den entsprechenden Phenylhydroxylaminen hydrieren lassen. Abgesehen von einer Verbindung mit einem 1,3,4-Oxadiazolsubstituenten wird die Hydrierung komplizierterer Systeme nach dieser Methode nicht beschrieben.

Der vorliegenden Erfindung liegt die Aufgabe zugrunde, ein Verfahren zur Herstellung von pyridylsubstituierten N-Phenylhydroxylaminverbindungen und zur Umlagerung dieser Verbindungen in Pyridyl-4-fluoranilinverbindungen zur Verfügung zu stellen, das einfach durchführbar ist und die gewünschten Verbindungen in hoher Ausbeute und Reinheit liefert.

Überraschenderweise wurde nun gefunden, daß diese Aufgabe gelöst wird, wenn man die Reduktion der entsprechenden Nitroverbindungen durch Hydrierung mit einem Platin- oder Palladiumkatalysator in Gegenwart einer am Stickstoffatom substituierten Morpholinverbindung durchführt.

Die vorliegende Erfindung betrifft daher ein Verfahren zur Herstellung von Verbindungen der Formel II 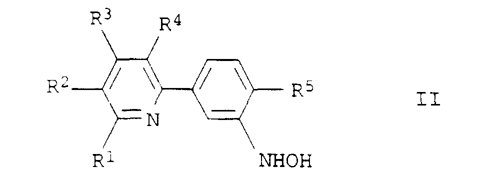 worin R¹, R³ und R⁴, die gleich oder verschieden sein können, für Wasserstoff, Halogen, Alkyl, Halogenalkyl, Alkoxyalkyl, Alkoxy, Alkoxyalkoxy, Hydroxyl, Halogenalkoxy, Alkylcarbonyloxy, Halogenalkylcarbonyloxy, Alkylsulfonyl, Halogenalkylsulfonyl, CO₂H, Alkoxycarbonyl, Alkoxyalkoxycarbonyl, Halogenalkoxycarbonyl, Alkylcarbonyl, Halogenalkylcarbonyl, Alkoxyalkylcarbonyl, CONH₂, Alkylaminocarbonyl, Dialkylaminocarbonyl, Pyrrolidinylcarbonyl, Piperidinylcarbonyl, Morpholinylcarbonyl, Amino, Alkylamino, Dialkylamino, Pyrrolidinyl, Piperidinyl, Morpholinyl, Alkylcarbonylamino, Halogenalkylcarbonylamino oder Alkylsulfonylamino stehen;
R² für Halogen, Alkyl, Halogenalkyl, Alkoxy oder Halogenalkoxy steht; oder
R¹ und R² oder R² und R³ zusammen eine Trimethylen- oder Tetramethylenkette bilden; und
R⁵ für Halogen, Hydroxyl, Trifluormethyl, Alkyl oder Alkoxy steht, wobei das Verfahren dadurch gekennzeichnet ist, daß man eine Verbindung der allgemeinen Formel III 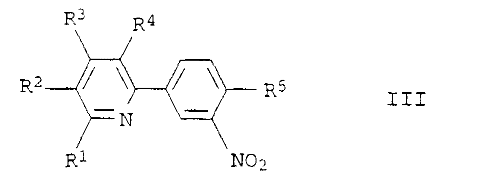 worin R¹ bis R⁵ die oben angegebenen Bedeutungen besitzen, in Anwesenheit eines Platinkatalysators oder eines mit Schwefel oder Selen dotierten Palladiumkatalysators und in Anwesenheit einer Morpholinverbindung der allgemeinen Formel IV 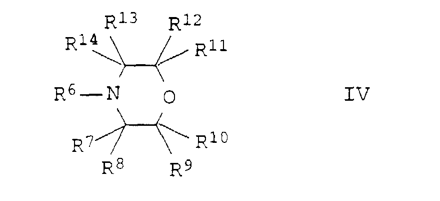 worin R⁶ für Alkyl steht und R⁷ bis R¹⁴, die gleich oder verschieden sein können, für ein Wasserstoffatom oder einen Alkylrest stehen, hydriert.

Weiter betrifft die Erfindung die Hydroxylaminverbindungen der Formel II, in der R¹ bis R⁵ die angegebenen Bedeutungen besitzen. Schließlich betrifft die Erfindung ein Verfahren zur Herstellung der Verbindungen der Formel I 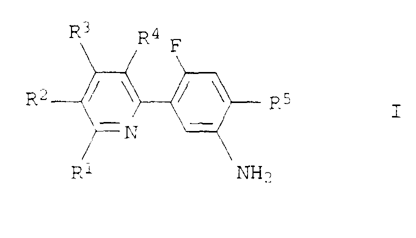 worin R¹ bis R⁵ die oben angegebenen Bedeutungen besitzen, das dadurch gekennzeichnet ist, daß man eine Verbindung der Formel II mit Fluorwasserstoff behandelt.

Im Rahmen der vorliegenden Erfindung steht Alkyl (auch in Kombination mit anderen Atomen, Gruppen oder Substituenten, wie Alkoxy, Halogenalkyl, Alkylcarbonyloxy, Alkylamino etc.) für geradkettige oder verzweigte Alkylgruppen, die vorzugsweise 1 bis 6 Kohlenstoffatome, insbesondere 1 bis 5 Kohlenstoffatome und besonders bevorzugt 1 bis 4 Kohlenstoffatome aufweisen. Beispiele für Alkylgruppen sind Methyl, Ethyl, n-Propyl, Isopropyl, n-Butyl, Isobutyl, sec-Butyl, n-Butyl, Pentyl und Isopentyl.

Eine Halogenalkylgruppe kann ein oder mehrere, insbesondere 1 bis 3 Halogenatome an einem oder mehreren Kohlenstoffatomen aufweisen. Besonders bevorzugt ist die Trifluormethylgruppe.

Halogen steht für Fluor, Chlor und Brom und insbesondere für Fluor oder Chlor.

Es ist überraschend und war nicht vorhersehbar, daß die Partialhydrierung von aromatischen Nitroverbindungen zu den entsprechenden Hydroxylaminderivaten nur in Anwesenheit von N-Alkylmorpholinen (N-substituierten Tetrahydro-1,4-oxazinen), die auch als Lösungsmittel dienen können, zu optimalen Ausbeuten und Selektivitäten führt, während nach der DE-A 24 55 238 organische Stickstoffbasen, wie Pyrrolidine, Piperidine, Aniline oder Pyridine als beste Lösungsmittel für diese Hydrierung empfohlen werden.

Da die als Reaktionsprodukte anfallenden Hydroxylaminderivate sehr labile Verbindungen sind, die sich nur schwer reinigen lassen und sich bei Einwirkung von Temperaturen > 100 °C relativ schnell zersetzen, ist es umso bedeutender, daß sie nach dem erfindungsgemäßen Verfahren bei weitgehend quantitativem Umsatz in hoher Reinheit anfallen, so daß eine aufwendige Reinigung durch Abtrennung von Ausgangsprodukt und Nebenprodukten, wie den entsprechenden Anilinen, Azoxybenzolen u.a. nicht erforderlich ist.

Die Herstellung der als Ausgangsprodukte benötigten Nitroaromaten ist beschrieben in der WO-A-95/02580.

Die nach dem Verfahren der Erfindung eingesetzten Katalysatoren enthalten Platin oder Palladium, vorzugsweise auf einem Kohleträger. Bei Verwendung eines Palladiumkatalysators muß dieser mit Schwefel oder Selen dotiert sein, um ausreichende Selektivität zu erhalten. Platin liefert beim Einsatz nach dem erfindungsgemäßen Verfahren auch ohne zusätzliche Dotierung ausgezeichnete Resultate. Die Katalysatoren können nach einem Reaktionscyclus abfiltriert und ohne spürbaren Aktivitätsverlust beim Folgeansatz wieder verwendet werden. Bei anderen Verfahren, die mit Zusätzen wie Dimethylsulfoxid, Dimethylaminopyridin oder org. Phosphorverbindungen arbeiten (EP-A- 86363, EP-A- 85890, EP-A- 147879), gibt es in der Regel einen schnellen Aktivitätsabfall durch Vergiftung der Katalysatoren.

Der Platin- bzw. Palladiumgehalt des Katalysators ist nicht kritisch und kann in weiten Grenzen variiert werden. Zweckmäßig ist ein Gehalt von 0,1 bis 15 Gew.-%, vorzugsweise von 0,5 bis 10 Gew.-%, bezogen auf das Trägermaterial Kohle. Die Menge des eingesetzten Platins oder Palladiums beträgt 0,001 bis 1 Gew.-%, bevorzugt 0,01 bis 0,1 Gew.-%, bezogen auf die Nitroverbindung. In der bevorzugten Ausführungsform einer diskontinuierlichen Hydrierung wird der Katalysator als Pulver eingesetzt. Von entscheidender Bedeutung für das Erzielen von sehr guten Ausbeuten ist die Anwesenheit der Morpholinverbindungen, da diese die Aktivität der Katalysatoren dergestalt beeinflussen, daß eine hohe Selektivität bei der Hydrierung von Nitroverbindungen zu den Hydroxylaminderivaten erreicht wird. Bevorzugte Morpholinverbindungen sind beispielsweise 4-Methylmorpholin, 4-Ethylmorpholin, 4-Propylmorpholin, 4-n-Butylmorpholin, 4-Isobutylmorpholin, 4-Tertiärbutylmorpholin, 4-n-Pentylmorpholin, 4-Isopentylmorpholin 2,4,6-Trimethylmorpholin, 2,3,4,5,6-Pentamethylmorpholin und 2,2,4,6,6-Pentamethylmorpholin.

In der Regel wird das Morpholin im Überschuß eingesetzt, d.h. das Gewichtsverhältnis von Morpholin zur Nitroverbindung ist größer als 1.

Die Temperatur für die Partialhydrierung liegt im allgemeinen im Bereich von zwischen - 20°C bis + 100°C, vorzugsweise - 5 bis + 50°C. Um Überhydrierungen zu vermeiden, wird bei der Temperatur, bei der die Hydrierung ausreichend schnell abläuft, ein Druck eingestellt, der zwischen Normaldruck und 10 bar Überdruck liegt. Normalerweise wird der Wasserstoff bei Normaldruck bzw. leicht erhöhtem Druck in den Hydrierreaktor eingegast. Vorzugsweise wird die Hydrierung ohne weitere Lösungs- bzw. Verdünnungsmittel, wie beispielsweise Alkohole oder Ether, durchgeführt.

Die Umlagerung der erhaltenen Hydroxylaminverbindungen in die gewünschten Pyridyl-4-fluoranilinverbindungen erfolgt durch Behandlung mit wasserfreiem Fluorwasserstoff. Dabei verwendet man die Reaktionspartner in solchen Mengen, daß das Molverhältnis von Fluorwasserstoff zu Verbindung der Formel II vorzugsweise im bereich von 10 bis 50 und insbesondere im Bereich von 20 bis 30 liegt.

Für die Durchführung der Umlagerung haben sich folgende Maßnahmen als besonders bevorzugt erwiesen:
a) die gewünschte Menge Fluorwasserstoff wird vorgelegt und das Hydroxylamin wird langsam zugegeben. Vorzugsweise wird das Hydroxylamin als Feststoff eingetragen. Wenn die Reaktionspartner in umgekehrter Reihenfolge in Kontakt gebracht werden, erhält man das Produkt in schlechterer Ausbeute und geringerer Reinheit.
b) das Hydroxylamin wird bei einer Temperatur im Bereich von -70 bis 0°C, insbesondere -40 bis -20°C, zugegeben. Nach beendeter Zugabe läßt man die Temperatur, gegebenenfalls unter Ausnutzung der Eigenreaktionswärme, ansteigen bis Rückflußbedingungen vorliegen.
c) Luftsauerstoff wird durch Arbeiten unter Inertgas, wie Stickstoff oder Argon, ausgeschlossen.
d) Die Reaktion wird ohne organische Lösungsmittel durchgeführt.

Die Reaktionszeit richtet sich nach der Reaktionstemperatur und den Edukten. Sie liegt im allgemeinen zwischen 5 und 24 Stunden.

Nach beendeter Reaktion erfolgt die Aufarbeitung in üblicher Weise. Es hat sich als zweckmäßig erwiesen, den Fluorwasserstoff mittels Erhitzen und Durchleiten von Stickstoff möglichst weitgehend auszutreiben. Der Rückstand wird dann in einen inerten organischen Lösungsmittel, beispielsweise einem chlorierten Lösungsmittel, wie Dichlormethan oder einem Ether, wie Diethylether, aufgenommen. Man gibt Wasser zu und stellt die Miscnung alkalisch, um die Aminbase freizusetzen, die dann mit einem geeigneten inerten Lösungsmittel, wie Diethylether oder Dichlormethan, extrahiert wird. Falls erforderlich kann eine Reinigung anhand üblicher Methoden, wie Umkristallisation und Chromatographie, erfolgen.

Die nachfolgenden Beispiele erläutern die Erfindung, ohne sie zu begrenzen.

### Beispiel 1

N-2-Chlor-5-(3-chlor-5-trifluormethyl-2-pyridyl)-phenylhydroxylamin

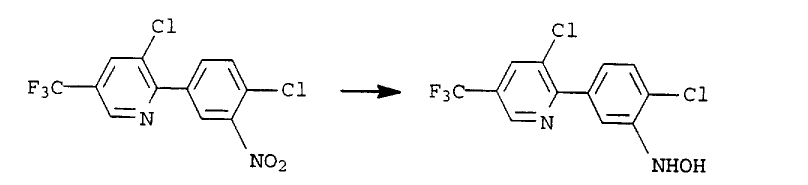

In eine 2-1-Hydrierapparatur gab man eine Lösung von 108 g (0,324 mol) 2-Chlor-5-(3-chlor-5-trifluormethyl-2-pyridyl)-nitrobenzol in 1,1 l N-Methylmorpholin und 4 g 5 gew-%iges Platin auf Kohle (Typ F 103 RS/W von Degussa). Nach dem Inertisieren mit Stickstoff wurde unter kräftigem Rühren bei 25-30°C und 0,1 bar Wasserstoffüberdruck solange Wasserstoff eingeleitet bis keine Aufnahme mehr erfolgte (ca. 6 h und 14,8 l Wasserstoffverbrauch).

Der Reaktionsaustrag wurde über Tierkohle abfiltriert, das Filtrat unter vermindertem Druck bei max. 60°C Badtemperatur eingedampft. Zum verbliebenen Rückstand wurden 100 g hochsiedendes Benzin (180 - 210°C) gegeben und mit Hilfe einer Vigreux-Kolonne wurde das Benzin /N-Methylmorpholin-Gemisch fraktioniert abdestilliert (Kp. 28-30°C bei 0,5 mbar). Die im Destillationssumpf ausgefallenen Kristalle wurden abgesaugt, mit n-Hexan gewaschen und getrocknet.

Man erhielt 103 g der Titelverbindung (entspricht einer Ausbeute von 98 %). Fp.: 103-105°C (unter Zers.)
250-MHz-¹H-NMR (DMSO-d₆, δ in ppm)
   9,05 (s, 1H), 8,7 (s, 1H), 8,55 (d, 1H); 8,5 (s, 1H); 7,57 (d, 1H); 7,41 (d, 1H); 7,15 (d, 1H)
analog wurde hergestellt:
   N-5-(3-chlor-5-trifluormethyl-2-pyridyl)-2-methoxy-phenylhydroxylamin 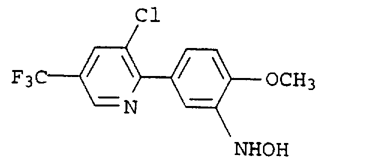
200-MHz-¹H-NMR (DMSO-d₆, δ in ppm)
   9,0 (s, 1H); 8,5 (breites s, 1H), 7,95 (breites s, 1H); 7,5 (s, 1H); 7,25 (d, 1H); 7,0 (d, 1H); 3,8 (s, 3H)

### Beispiel 2

2-Chlor-4-fluor-5-(3-chlor-5-trifluormethyl-2-pyridyl)-4-fluoranilin

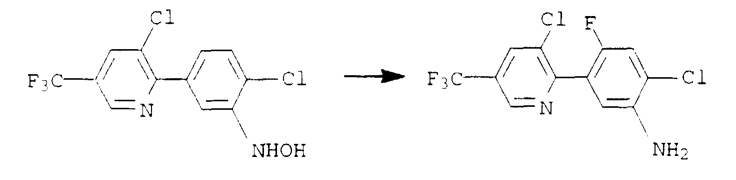

Ein 1-l-Teflon-Reaktionsgefäß (ausgerüstet mit Rückflußkühler, Gaseinleitungsrohr, Innentemperaturmessung) wurde nach dem Inertisieren mit Stickstoff in einem Trockeneis-Bad gekühlt, der Kühlkreislauf im Rückflußkühler wurde auf -15°C eingestellt. Man begann mit dem Einkondensieren des Fluorwasserstoffs, dabei lag die Innentemperatur im Bereich -49 bis -66°C. Nachdem insgesamt 317 g (15,9 mol) Fluorwasserstoff eingebracht worden waren, wurde das HF-Gaseinleitungsrohr entfernt und 162 g (0,5 mol) N-2-Chlor-5-(3-chlor-5-trifluormethyl-2-pyridyl)-phenylhydroxylamin (Beispiel 1) wurden portionsweise in 30 min eingetragen. Dabei stieg die Innentemperatur auf -48°C an. Anschließend entfernte man das Kältebad, hob die Temperatur des Kühlkreislaufs im Abgaskühler auf 25°C an und ließ das Reaktionsgemisch auf Umgebungstemperatur kommen. Nach 3 Stunden waren 23°C erreicht. In den anschließenden 2 Stunden erhöhte sich die Innentemperatur auf maximal 36°C.

10 Stunden nach Zudosierungsende wurde die Temperatur im Kühlkreislauf auf 30°C erhöht und das austretende Fluorwasserstoffgas in einen Waschturm mit Kalilauge geleitet. Später wurde das Reaktionsgefäß zusätzlich mit Stickstoff gespült.

Danach wurde das Reaktionsgefäß geöffnet und das dunkle, flüssige Reaktionsgemisch wurde portionsweise in 400 g Eis unter kräftigem Rühren eingetragen. Es wurde mit insgesamt 300 ml Dichlormethan nachgespült. Die zweiphasige Mischung wurde dann mit 25%iger Ammoniaklösung auf pH = 10 eingestellt. Nach Abtrennen der organischen Phase extrahierte man die wäßrige Phase noch zweimal mit je 350 ml Dichlormethan, vereinigte die organischen Phasen, trocknete über Magnesiumsulfat und verdampfte schließlich das Lösemittel unter vermindertem Druck.

Man erhielt 155 g eines festen Rückstand, der aus 780 ml n-Hexan umkristallisiert wurde, wobei man im Eisbad abkühlte.
Ausbeute: 140,2 g (86 % d.Th.) der Titelverbindung
Reinheit nach GC: > 99 Flächen-%.
Fp.: 94-95°C
270-MHz-¹H-NMR (CDCl₃, δ in ppm)
   8,85 (s, 1H); 8,05 (s, 2H), 7,15 (d, 1H); 6,81 (d, 1H); 4,5 (s, 2H)

## Patentansprüche

1. N-Phenylhydroxylaminverbindungen der Formel II 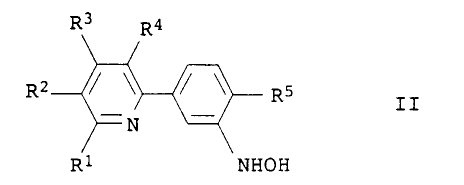 worin
R¹, R³ und R⁴, die gleich oder verschieden sein können, für Wasserstoff, Halogen, Alkyl, Halogenalkyl, Alkoxyalkyl, Alkoxy, Alkoxyalkoxy, Hydroxyl, Halogenalkoxy, Alkylcarbonyloxy, Halogenalkylcarbonyloxy, Alkylsulfonyl, Halogenalkylsulfonyl, CO₂H, Alkoxycarbonyl, Alkoxyalkoxycarbonyl, Halogenalkoxycarbonyl, Alkylcarbonyl, Halogenalkylcarbonyl, Alkoxyalkylcarbonyl, CONH₂, Alkylaminocarbonyl, Dialkylaminocarbonyl, Pyrrolidinylcarbonyl, Piperidinylcarbonyl, Morpholinylcarbonyl, Amino, Alkylamino, Dialkylamino, Pyrrolidinyl, Piperidinyl, Morpholinyl, Alkylcarbonylamino, Halogenalkylcarbonylamino oder Alkylsulfonylamino stehen;
R² für Halogen, Alkyl, Halogenalkyl, Alkoxy oder Halogenalkoxy steht; oder
R¹ und R² oder R² und R³ zusammen eine Trimethylen- oder Tetramethylenkette bilden; und
R⁵ für Halogen, Hydroxyl, Trifluormethyl, Alkyl oder Alkoxy steht.

2. Verbindungen nach Anspruch 1 der Formel II, worin R¹, R³ und R⁴, die gleich oder verschieden sein können, für Wasserstoff, Halogen, Alkyl, Halogenalkyl, Alkoxy, Hydroxyl, Halogenalkoxy oder Alkylsulfonyl stehen.

3. Verbindungen nach Anspruch 2 der Formel II, worin R¹, R³ und R⁴, die gleich oder verschieden sein können, für Wasserstoff, Halogen, Alkyl oder Halogenalkyl stehen.

4. Verbindungen nach einem der vorhergehenden Ansprüche der Formel II, worin R² für Halogen oder Halogenalkyl steht.

5. Verbindungen nach einem der vorhergehenden Ansprüche der Formel II, worin R⁵ für Halogen oder Alkoxy steht.

6. Verbindungen nach Anspruch 1 der Formel II, worin R¹ und R³ für Wasserstoff stehen; R² für Halogen oder Halogenalkyl steht; R⁴ für Alkyl oder Halogen steht; und R⁵ für Halogen oder Alkoxy steht.

7. Verfahren zur Herstellung der Verbindungen der Formel II nach einem der Ansprüche 1 bis 6, dadurch gekennzeichnet, daß man eine Verbindung der allgemeinen Formel III 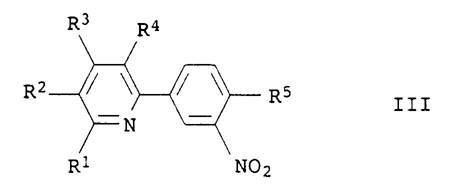 worin R¹ bis R⁵ die in einem der Ansprüche 1 bis 6 angegebenen Bedeutungen besitzen, in Anwesenheit eines Platinkatalysators oder eines mit Schwefel oder Selen dotierten Palladiumkatalysators und in Anwesenheit einer Morpholinverbindung der allgemeinen Formel IV 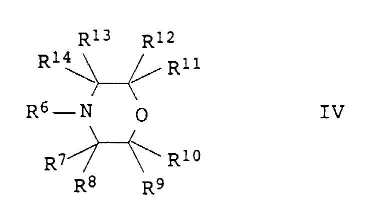 worin R⁶ für Alkyl steht und R⁷ bis R¹⁴, die gleich oder verschieden sein können, für ein Wasserstoffatom oder einen Alkylrest stehen, hydriert.

8. Verfahren zur Herstellung der Verbindungen der Formel I 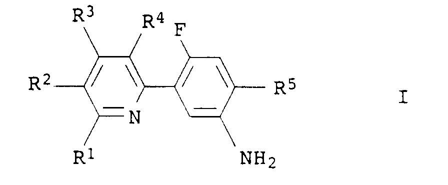 worin R¹ bis R⁵ die in einem der Ansprüche 1 bis 6 angegebenen Bedeutungen besitzen, dadurch gekennzeichnet, daß man eine Verbindung der Formel II mit Fluorwasserstoff behandelt.

9. Verfahren nach Anspruch 8, dadurch gekennzeichnet, daß das Molverhältnis von Fluorwasserstoff zu Verbindung der Formel II im Bereich von 10 bis 50 liegt.

10. Verfahren nach Anspruch 8 oder 9, dadurch gekennzeichnet, daß man den Fluorwasserstoff vorlegt und die Verbindung der Formel II allmählich zugibt.

11. Verfahren nach Anspruch 8, dadurch gekennzeichnet, daß die Zugabe der Verbindung der Formel II bei einer Temperatur im Bereich von -70 bis -40 °C erfolgt.

12. Verfahren nach einem der Ansprüche 8 bis 11, dadurch gekennzeichnet, daß man die Verbindungen der Formel II herstellt, indem man eine Verbindung der allgemeinen Formel III 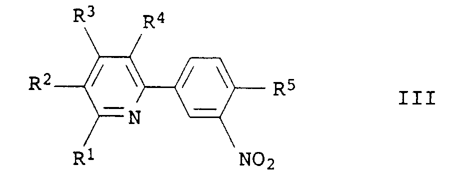 worin R¹ bis R⁵ die in einem der Ansprüche 1 bis 6 angegebenen Bedeutungen besitzen, in Anwesenheit eines Platinkatalysators oder eines mit Schwefel oder Selen dotierten Palladiumkatalysators und in Anwesenheit einer Morpholinverbindung der allgemeinen Formel IV 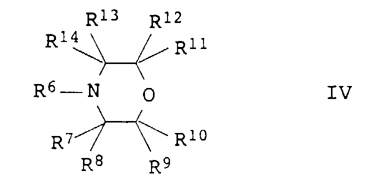 worin R⁶ für Alkyl steht und R⁷ bis R¹⁴, die gleich oder verschieden sein können, für ein Wasserstoffatom oder einen Alkylrest stehen, hydriert.

## Claims

1. An N-phenylhydroxylamine compound of the formula II 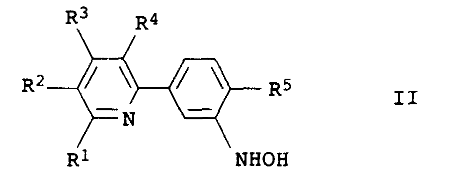 where
R¹, R³ and R⁴, which can be identical or different, are hydrogen, halogen, alkyl, haloalkyl, alkoxyalkyl, alkoxy, alkoxyalkoxy, hydroxyl, haloalkoxy, alkylcarbonyloxy, haloalkylcarbonyloxy, alkylsulfonyl, haloalkylsulfonyl, CO₂H, alkoxycarbonyl, alkoxyalkoxycarbonyl, haloalkoxycarbonyl, alkylcarbonyl, haloalkylcarbonyl, alkoxyalkylcarbonyl, CONH₂, alkylaminocarbonyl, dialkylaminocarbonyl, pyrrolidinylcarbonyl, piperidinylcarbonyl, morpholinylcarbonyl, amino, alkylamino, dialkylamino, pyrrolidinyl, piperidinyl, morpholinyl, alkylcarbonylamino, haloalkylcarbonylamino or alkylsulfonylamino;
R² is halogen, alkyl, haloalkyl, alkoxy or haloalkoxy; or
R¹ and R² or R² and R³ together form a trimethylene or tetramethylene chain; and
R⁵ is halogen, hydroxyl, trifluormethyl, alkyl or alkoxy.

2. A compound as claimed in claim 1, of the formula II, where R¹, R³ and R⁴, which can be identical or different, are hydrogen, halogen, alkyl, haloalkyl, alkoxy, hydroxyl, haloalkoxy or alkylsulfonyl.

3. A compound as claimed in claim 2, of the formula II, where R¹, R³ and R⁴, which can be identical or different, are hydrogen, halogen, alkyl or haloalkyl.

4. A compound as claimed in one of the preceding claims, of the formula II, where R² is halogen or haloalkyl.

5. A compound as claimed in one of the preceding claims, of the formula II, where R⁵ is halogen or alkoxy.

6. A compound as claimed in claim 1, of the formula II, where R¹ and R³ are hydrogen, R² is halogen or haloalkyl; R⁴ is alkyl or halogen; and R⁵ is halogen or alkoxy.

7. A process for preparing a compound of the formula II as claimed in one of claims 1 to 6, which comprises hydrogenating a compound of the general formula III 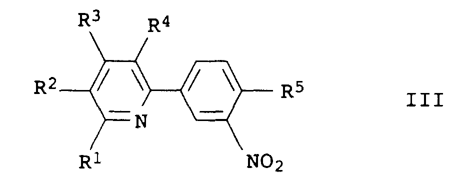 where R¹ to R⁵ have the meanings indicated in one of claims 1 to 6, in the presence of a platinum catalyst or of a sulfur- or selenium-doped palladium catalyst and in the presence of a morpholine compound of the general formula IV 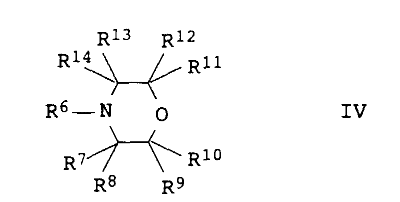 where R⁶ is alkyl and R⁷ to R¹⁴, which can be identical or different, are a hydrogen atom or an alkyl radical.

8. A process for preparing a compound of the formula I 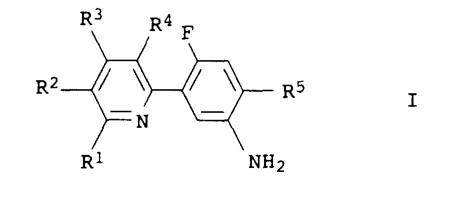 where R¹ to R⁵ have the meanings indicated in one of claims 1 to 6, which comprises treating a compound of the formula II with hydrogen fluoride.

9. A process as claimed in claim 8, wherein the molar ratio of hydrogen fluoride to compound of the formula II is in the range from 10 to 50.

10. A process as claimed in claim 8 or 9, wherein the hydrogen fluoride is initially introduced and the compound of the formula II is gradually added.

11. A process as claimed in claim 8, wherein the addition of the compound of the formula II is carried out in the range from -70 to -40°C.

12. A process as claimed in one of claims 8 to 11, wherein a compound of the formula II is prepared by hydrogenating a compound of the general formula III 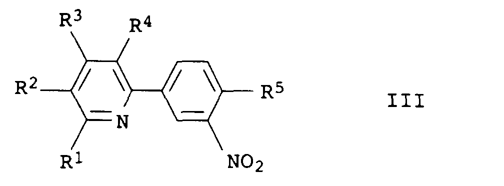 where R¹ to R⁵ have the meanings indicated in one of claims 1 to 6, in the presence of a platinum catalyst or a sulfur- or selenium-doped palladium catalyst and in the presence of a morpholine compound of the general formula IV 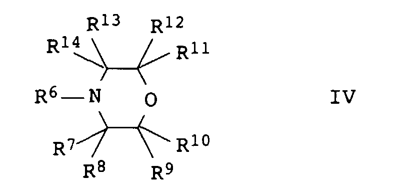 where R⁶ is alkyl and R⁷ to R¹⁴, which can be identical or different, are a hydrogen atom or an alkyl radical.

## Revendications

1. Dérivés N-phénylhydroxylaminés de formule II 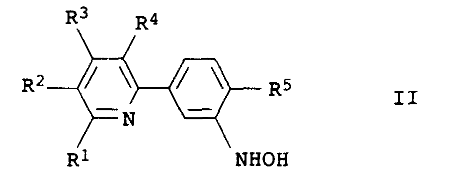 dans laquelle
R¹, R³ et R⁴, ayant des significations identiques ou différentes, représentent chacun l'hydrogène, un halogène, un groupe alkyle, halogénoalkyle, alcoxyalkyle, alcoxy, alcoxyalcoxy, hydroxy, halogénoalcoxy, alkylcarbonyloxy, halogénoalkylcarbonyloxy, alkylsulfonyle, halogénoalkylsulfonyle, CO₂H, alcoxycarbonyle, alcoxyalcoxycarbonyle, halogénoalcoxycarbonyle, alkylcarbonyle, halogénoalkylcarbonyle, alcoxyalkylcarbonyle, CONH₂, alkylaminocarbonyle, dialkylaminocarbonyle, pyrrolidinylcarbonyle, pipéridinylcarbonyle, morpholinylcarbonyle, amino, alkylamino, dialkylamino, pyrrolidinyle, pipéridinyle, morpholinyle, alkylcarbonylamino, halogénoalkylcarbonylamino ou alkylsulfonylamino ;
R² représente un halogène, un groupe alkyle, halogénoalkyle, alcoxy ou halogénoalcoxy ;
ou bien
R¹ et R² ou R² et R³ représentent ensemble une chaîne triméthylène ou tétraméthylène ; et
R⁵ représente un halogène, un groupe hydroxy, trifluorométhyle, alkyle ou alcoxy.

2. Composés selon la revendication 1, de formule II dans laquelle R¹, R³ et R⁴, ayant des significations identiques ou différentes, représentent chacun l'hydrogène, un halogène, un groupe alkyle, halogénoalkyle, alcoxy, hydroxy, halogénoalcoxy ou alkylsulfonyle.

3. Composés selon la revendication 2, de formule II dans laquelle R¹, R³ et R⁴, ayant des significations identiques ou différentes, représentent chacun l'hydrogène, un halogène, un groupe alkyle ou halogénoalkyle.

4. Composés selon l'une des revendications qui précèdent, de formule II dans laquelle R² représente un halogène ou un groupe halogénoalkyle.

5. Composés selon l'une des des revendications qui précèdent, de formule II dans laquelle R⁵ représente un halogène ou un groupe alcoxy.

6. Composés selon la revendication 1, de formule II dans laquelle R¹ et R³ représentent l'hydrogène ; R² représente un halogène ou un groupe halogénoalkyle ; R⁴ représente un groupe alkyle ou un halogène ; et R⁵ représente un halogène ou un groupe alcoxy.

7. Procédé de préparation des composés de formule II selon l'une des revendications 1 à 6, caractérisé par le fait que l'on hydrogène un composé de formule générale III 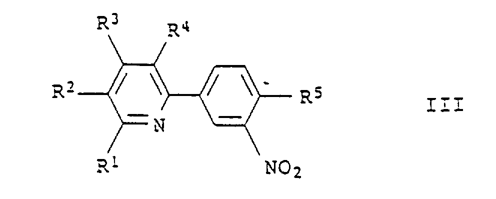 dans laquelle R¹ à R⁵ ont les significations indiquées dans l'une des revendications 1 à 6, en présence d'un catalyseur au platine ou d'un catalyseur au palladium dopé par du soufre ou du sélénium et en présence d'un dérivé de la morpholine répondant à la formule générale IV 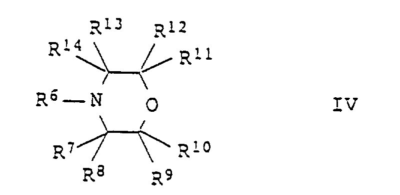 dans laquelle R⁶ représente un groupe alkyle et R⁷ à R¹⁴, ayant des significations identiques ou différentes, représentent chacun un atome d'hydrogène ou un groupe alkyle.

8. Procédé de préparation des composés de formule I 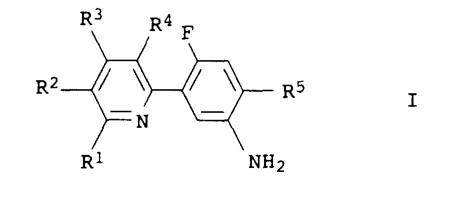 dans laquelle R¹ à R⁵ ont les significations indiquées dans l'une des revendications 1 à 6, caractérisé par le fait que l'on traite un composé de formule II par le fluorure d'hydrogène.

9. Procédé selon la revendication 8, caractérisé par le fait que le rapport molaire entre le fluorure d'hydrogène et le composé de formule II se situe dans l'intervalle de 10 à 50.

10. Procédé selon les revendications 8 ou 9, caractérisé par le fait l'on ajoute peu à peu le composé de formule II au fluorure d'hydrogène.

11. Procédé selon la revendication 8, caractérisé par le fait que l'on ajoute le composé de formule II à des températures dans l'intervalle de -70 à -40°C.

12. Procédé selon l'une des revendications 8 à 11, caractérisé par le fait que l'on prépare les composés de formule II en hydrogénant un composé de formule générale III 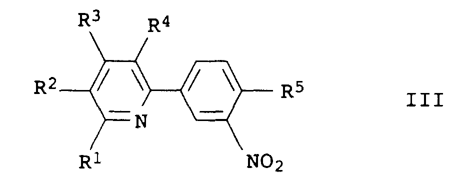 dans laquelle R¹ à R⁵ ont les significations indiquées dans l'une des revendications 1 à 6, en présence d'un catalyseur au platine ou d'un catalyseur au palladium dopé par du soufre ou du sélénium et en présence d'un dérivé de la morpholine répondant à la formule générale IV 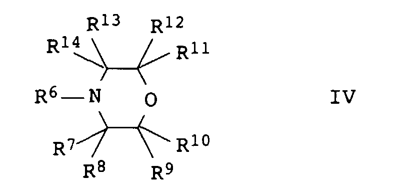 dans laquelle R⁶ représente un groupe alkyle et R⁷ à R¹⁴, ayant des significations identiques ou différentes, représentent chacun un atome d'hydrogène ou un groupe alkyle.
